Europäisches Patentamt

European Patent Office

Office européen des brevets

(11) Veröffentlichungsnummer: **0 263 259 B1**

(12) **EUROPÄISCHE PATENTSCHRIFT**

(45) Veröffentlichungstag der Patentschrift: **19.06.91**

(21) Anmeldenummer: **87111457.5**

(22) Anmeldetag: **07.08.87**

Die Akte enthält technische Angaben, die nach dem Eingang der Anmeldung eingereicht wurden und die nicht in dieser Patentschrift enthalten sind.

(51) Int. Cl.⁵: **C07C 2/48**, C07C 15/14, C07C 29/44, C07C 33/26, C07C 41/30, C07C 43/164, C07C 43/166, B01J 31/18

(54) Verfahren und Katalysatorsystem zur Trimerisierung von Acetylen und Acetylenverbindungen.

(30) Priorität: **29.09.86 DE 3633033**

(43) Veröffentlichungstag der Anmeldung:
**13.04.88 Patentblatt 88/15.**

(45) Bekanntmachung des Hinweises auf die Patenterteilung:
**19.06.91 Patentblatt 91/25**

(84) Benannte Vertragsstaaten:
**BE CH DE FR GB IT LI NL**

(56) Entgegenhaltungen:
**DD-A- 207 704**
**DD-A- 240 198**
**DE-A- 2 046 200**
**DE-A- 2 056 555**
**DE-C- 1 159 951**

**CHEMICAL ABSTRACTS, Band 93, No.3, 21. Juli 1980, Zusammenfassungsnr. 25811y; J.A.K. DU PLESSIS et al.: "Homogeneous catalysis in the cyclization of acetylene"**

**CHEMICAL ABSTRACTS, Band 64, Nr. 11, 23.**

Mai 1966, Zusammenfassungsnr. 15986b, Spalte 2; C.H. BAMFORD et al.: **"Tetrakis(triphenyl phosphite)nickel(0)as an initiator of polymerization at room temperature"**

(73) Patentinhaber: **HÜLS AKTIENGESELLSCHAFT Patentabteilung / PB 15 - Postfach 13 20 W-4370 Marl 1(DE)**

(72) Erfinder: **Thelen, Gerhard, Dr. Sepp-Herberger-Strasse 62 W-4405 Nottuln(DE)**
Erfinder: **Voges, Heinz-Werner, Dr. Im Gorden 45 W-4270 Dorsten(DE)**

## Beschreibung

Es ist bekannt, daß man Acetylen und Acetylenverbindungen zu Benzol und den entsprechenden Benzolderivaten cyclisieren kann. Nach Reppe entsteht aus Propargylalkohol in Gegenwart von Ni-$(CO)_2$ $[P(C_6H_5)_3]_2$ als Katalysator in nahezu quantitativer Ausbeute ein Gemisch aus 1,3,5- und 1,2,4-Tris(hydroxymethyl)benzol. Diese Umsetzung verläuft so stürmisch, daß man sie praktisch nur in starker Verdünnung und bei langsamer Zugabe der Acetylenkomponente durchführen kann. Selbst dann bleibt das Produkt durch schwierig abtrennbare Nebenprodukte verunreinigt. Ein weiterer Nachteil des Katalysators besteht darin, daß sich außer Acetylen nur wenige Acetylenverbindungen trimerisieren lassen.

In der DE-PS 11 59 951 werden carbonylfreie Phosphit oder Thiophosphit enthaltende Nickel(0)-Komplex-Verbindungen beschrieben, die durch Umsetzung von Nickel(0)-bis-acrylnitril, Nickel(0)-bis-acrolein oder Nickel(0)-bis-durochinon synthetisiert werden. Mit diesen Verbindungen lassen sich unter milden Reaktionsbedingungen teilweise ausgezeichnete Ausbeuten erzielen. Besonders gute Ergebnisse werden mit Arylphosphit-Liganden erhalten, die in o-Stellung zum Sauerstoffatom großvolumige Substituenten tragen. Der Nachteil dieses Verfahrens beruht darauf, daß die verwendeten Nickel-Verbindungen letztlich aus $Ni(CO)_4$ präpariert werden müssen.

In den Patentschriften DE-PS 20 46 200 und DE-PS 20 56 555 sind für die Trimerisierung von 3-Methylbut-1-in-3-ol die Verwendung von Nickeltetracarbonyl in Kombination mit Triarylphospiten angegeben, wobei auch in diesem Falle ebenfalls ausgezeichnete Ergebnisse erzielt werden, wenn in o-Stellung sterisch anspruchsvolle Substituenten vorhanden sind.

Unter den heutigen Sicherheitsaspekten kommen Verfahren unter Verwendung von Nickeltetracarbonyl für die industrielle Nutzung nicht mehr in Frage. Es besteht daher ein Interesse an einem Verfahren, bei dem man eine leicht handhabbare, sicherheitstechnisch unbedenkliche Katalysatorkomponente einsetzen kann.

Die sich hieraus ergebende Aufgabe wird erfindungsgemäß entsprechend den Angaben der Patentansprüche gelöst.

Eine Carbonyl-Ligand-freie Nickel(0)-Verbindung ist das Tetrakis(triphenylphosphit)nickel. Von diesem Nickelkomplex ist einerseits die geringe Luft- und Temperaturempfindlichkeit, andererseits die Reaktionsträgheit und die damit verbundene begrenzte Verwendung in der Nickelchemie bekannt.

Es wurde nun überraschend gefunden, daß man Acetylenverbindungen in Gegenwart von Tetrakis(triphenylphosphit)nickel(0) katalytisch trimerisieren kann, wenn mindestens eine weiterere Phosphit-Verbindung anwesend ist und die Umsetzung gegebenenfalls in einem inerten Lösemittel und bei einer Temperatur von 20 bis 120 °C, vorzugsweise 50 bis 100 °C, insbesondere 70 bis 90 °C, durchgeführt wird.

Als inerte Lösemittel sind aliphatische, aromatische und araliphatische Kohlenwasserstoffe, wie z. B. Hexan, n-Oktan, Isooktan, Cyclohexan, Cyclooktan, Benzol, Toluol und Xylol geeignet.

Mit diesem Katalysatorsystem lassen sich die verschiedenartigsten Acetylenverbindungen, die neben der CC-Dreifachbindung zusätzlich mit gesättigten oder ungesättigten Gruppen substituiert sein können, trimerisieren. Von den geeigneten Verbindungen seien beispielsweise genannt: Methylacetylen, Phenylacetylen, 3-Methylbut-3-en-1-in, 4-Methoxybut-3-en-1-in, Propargylalkohol, Propargylethylether, 3-Methylbut-1-in-3-ol, 3-Methylpent-1-in-3-ol, But-2-in-1,4-diol.

Die besten Ergebnisse erzielt man durch Kombination von Tetrakis(triphenylphosphit)nickel(0) mit solchen Phosphit-Verbindungen, vorzugsweise Phosphorigsäureestern, die in o-Stellung zum Heteroatom großvolumige Substituenten tragen. Als Phosphite sind auch Thiophosphite geeignet.

Phosphite dieser Art sind z. B. Tris(2-isopropyl-5-methylphenyl) phosphit, Tris(2,6-dimethylphenyl)-phosphit, Tris(2-tert.-butyl-5-methylphenyl)phosphit, Tris(2-sek.-butylphenyl)phosphit, Tris(2-tert.-butyl-4-methylphenyl)phosphit, Tris(2,4-ditert.-butylphenyl)phosphit, Tris(2-tert.-butylphenyl)-phosphit, Tris(2-diphenylyl)phosphit.

Man führt die Umsetzung der Nickel(0)-Verbindung mit den Phosphiten zur Herstellung des Katalysators vorzugsweise in einem inerten Lösemittel und bei einer Temperatur von 20 °C bis 120 °C, vorzugsweise 50 bis 100 °C, insbesondere von 70 bis 90 °C, durch, indem beide Komponenten in einem Molverhältnis von 1 : 1 bis 1 : 5, vorzugsweise 1: 1,5 bis 1 : 2,5, bevorzugt in einer Inertgasatmosphäre, einige Zeit unter Rühren gemischt werden.

Geeignete Lösemittel sind aliphatische sowie aromatische und araliphatische Kohlenwasserstoffe, wie z. B. n-Hexan, n-Oktan, Isooktan, Cyclohexan, Cyclooktan, Benzol, Toluol und Xylol.

Man führt das erfindungsgemäße Verfahren durch, indem man die Ausgangsverbindung mit dem Katalysator in Berührung bringt. Bevorzugt wendet man 1 bis 10 Gewichtsprozent Katalysator, insbesondere 5 bis 8 Gewichtsprozent, bezogen auf das Edukt, an. Die Umsetzung wird bei einer Temperatur von 20 bis 120 °C, vorzugsweise von 50 bis 100 °C, insbesondere von 70 bis 90 °C, durchgeführt, bevorzugt in einem Lösemittel, das auch für die Bereitung des Katalysators eingesetzt wurde. Die Herstellung des Katalysators und die Trimerisierung führt man vorzugsweise in einer Stufe durch.

Man arbeitet im allgemeinen unter Atmosphärendruck, höhere Drücke werden insbesondere dann angewandt, wenn die Reaktionstemperatur über dem Siedepunkt der Acetylenverbindungen und/oder des gewählten Lösemittels liegt.
Die Umsetzung kann diskontinuierlich und kontinuierlich durchgeführt werden.
Die nach dem Verfahren erhaltenen Produkte sind wertvolle Zwischenprodukte zur Herstellung polyfunktioneller Benzolabkömmlinge.

Beispiel 1

In einem Rundkolben werden 0,5 g Tetrakis-(triphenylphosphit)nickel(0), 0,3 g Tris(2-tert.-butylphenyl)phosphit und 10 ml 3-Methylbut-1-in-3-ol in 10 ml Toluol gelöst und unter Rühren auf 100 °C erwärmt. Zur Aufarbeitung kühlt man nach 5 Stunden auf Raumtemperatur ab, filtriert den auskristallisierten Feststoff, wäscht mit Toluol und Ether und trocknet im Vakuum. Nach dem NMR-Spektrum besteht das Produkt aus 1,3,5-Tris(alpha-hydroxyisopropyl)benzol. Die Ausbeute beträgt 60 % der Theorie.

Beispiel 2

In der im Beispiel 1 beschriebenen Weise werden 0,5 g Tetrakis(triphenylphosphit)nickel(0), 0,18 g Tris(2-sek.-butylphenyl)phosphit und 10 ml Methylbutinol in 15 ml n-Heptan bei Reaktionstemperaturen von 90 °C umgesetzt. Die Ausbeute beträgt 60 % der Theorie.

Beispiel 3

0,5 g Tetrakis(triphenylphosphit)nickel(0) und 0,18 g Tris(2-sek.-butylphenyl)phosphit werden in 15 ml n-Heptan auf Rückflußtemperatur (98 °C) erwärmt. Zu dieser Mischung werden innerhalb einer Stunde 29 g Methylbutinol dosiert. Nach 5 Stunden Reaktionsdauer läßt man abkühlen und arbeitet, wie im Beispiel 1 angegeben, auf. Die Ausbeute beträgt 41 % der Theorie.

Beispiel 4

In einem Rundkolben werden 0,5 g Tetrakis-(triphenylphosphit)nickel(0), 0,18 g Tris(2-sek.-butylphenyl)phosphit und 10 ml Phenylacetylen unter Rühren auf 100 °C erwärmt. Zur Aufarbeitung destilliert man unter vermindertem Druck unumgesetztes Phenylacetylen ab und kristallisiert den Rückstand aus Ethanol um. Das Produkt besteht aus 1,3,5-Triphenylbenzol. Die Ausbeute beträgt 40 % der Theorie.

Vergleichsbeispiel A

Führt man die Reaktion, wie im Beispiel 1 beschrieben, ohne Zusatz des Tris(2-tert.-butylphenyl)phosphits durch, so läßt sich nach dem Abkühlen kein 1,3,5-Tris(alpha-hydroxyisopropyl)benzol isolieren.

**Ansprüche**

1. Verfahren zur Trimerisierung von Acetylen und Acetylenverbindungen zu Benzol bzw. den entsprechenden Benzolderivaten,
dadurch gekennzeichnet,
daß man Acetylen bzw. Acetylenverbindungen mit Hilfe eines Katalysators aus Tetrakis-(triphenylphosphit)nickel(0) und mindestens einer weiteren Phosphit-Verbindung bei einer Temperatur von 20 bis 120 °C, gegebenenfalls in einem inerten Lösemittel, umsetzt.

2. Verfahren nach Anspruch 1,
dadurch gekennzeichnet,
daß man als Acetylenverbindungen Methylacetylen, Phenylacetylen, 3-Methylbut-3-en-1-in, 4-Methoxybut-3-en-1-in, Propargylalkohol, Propargylethylether, 3-Methylbut-1-in-3-ol, 3-Methylpent-1-in-3-ol oder But-2-in-1,4-diol einsetzt.

3. Verfahren nach Anspruch 1 und 2,
dadurch gekennzeichnet,
daß man als weitere Phosphit-Verbindung Tris-(2-isopropyl-5-methylphenyl)phospit, Tris(2,6-dimethylphenyl)-phosphit, Tris(2-tert.-butyl-5-methylphenyl)phosphit, Tris(2-sek.-butylphenyl)phosphit, Tris(2-tert.-butyl-4-methylphenyl)phosphit, Tris(2,4-ditert.-butylphenyl)phosphit, Tris(2-tert.-butylphenyl)-phosphit oder Tris(2-diphenylyl)phosphit einsetzt.

4. Verfahren nach einem der Ansprüche 1 bis 3,
dadurch gekennzeichnet,
daß man die Umsetzung bei einer Temperatur von 50 bis 100 °C, vorzugsweise 70 bis 90 °C, durchführt.

5. Verfahren nach einem der Ansprüche 1 bis 4,
dadurch gekennzeichnet,
daß man das Tetrakis(triphenylphosphit)nickel-(0) und weitere Phosphit-Verbindungen im Molverhältnis von 1 : 1 bis 1 : 5, vorzugsweise von 1 : 1,5 bis 1 : 2,5, einsetzt.

6. Verfahren nach einem der Ansprüche 1 bis 5,
dadurch gekennzeichnet,

daß man zur Katalysatorherstellung und/oder zur Trimerisierung aliphatische, aromatische und/oder araliphatische Kohlenwasserstoffe, wie Hexan, n-Oktan, Isooktan, Cyclohexan, Cyclooktan, Benzol, Toluol oder Xylol, einsetzt.

7. Verfahren nach einem der Ansprüche 1 bis 6, dadurch gekennzeichnet, daß man den Katalysator bei Temperaturen von 20 bis 120 °C, vorzugsweise von 50 bis 100 °C, herstellt.

8. Katalysatorsystem zur Trimerisierung von Acetylen und Acetylenverbindungen, gekennzeichnet durch Tetrakis(triphenylphosphit)nickel(0) und mindestens eine weitere Phosphit-Verbindung, ausgewählt aus Tris(2-isopropyl-5-methylphenyl)phosphit, Tris(2,6-dimethylphenyl)phosphit, Tris(2-tert.-butyl-5-methylphenyl)phosphit, Tris-(2-sek.-butylphenyl)phosphit, Tris(2-tert.-butyl-4-methylphenyl)phosphit, Tris(2,4-ditert.-butylphenyl)-phosphit, Tris(2-tert.-butylphenyl)-phosphit oder Tris(2-diphenylyl)-phosphit.

9. Katalysatorsystem nach Anspruch 8, gekennzeichnet durch ein Molverhältnis von Tetrakis-(triphenylphosphit)nickel(0) zu weiterer Phosphit-Verbindung von 1 : 1 bis 1 : 5, vorzugsweise von 1 : 1,5 bis 1 : 2,5.

## Claims

1. A process for the trimerization of acetylene and acetylene compounds to give benzene or the corresponding benzene derivatives, characterized in that acetylene or acetylene compounds is or are reacted by means of a catalyst of tetrakis(triphenylphosphite)nickel(O) and at least one further phosphite compound at a temperature of 20 to 120 °C, if necessary in an inert solvent.

2. A process according to claim 1, characterized in that the acetylene compounds used are methyl acetylene, phenyl acetylene, 3-methylbut-3-en-1-ine, 4-methoxybut-3-en-1-ine, propargylalcohol, propargylethyl ether, 3-methylbut-1-in -3-ol, 3-methylpent-1-in -3-ol or but-2-in -1, 4-diol.

3. A process according to claims 1 and 2, characterized in that use is made as the further phosphite compound of tris(2-isopropyl-5-methylphenyl)phosphite, tris(2,6-dimethylphenyl)-phosphite, tris(2-tert.-butyl-5-methylphenyl)phosphite, tris(2-sec.-butylphenyl)-

phosphite, tris(2-tert.-butyl-4-methylphenyl)-phosphite, tris(2,4-ditert.-butylphenyl)-phosphite, tris(2-tert.-butylphenyl)phosphite or tris(2-diphenylyl)phosphite.

4. A process according to one of claims 1 to 3, characterized in that the reaction is performed at a temperature of 50 to 100 °C, preferably 70 to 90 °c.

5. A process according to one of claims 1 to 4, characterized in that the the molar ratio between the tetrakis(triphenylphosphite)nickel(O) and the further phosphite compounds is 1 : 1 to 1 : 5, preferably 1 : 1.5 to 1 : 2.5.

6. A process according to one of claims 1 to 5, characterized in that for making the catalyst and/or for trimerization use is made of aliphatic, aromatic and/or araliphatic hydrocarbons, such as hexane, n-octane, isooctane, cyclohexane, cyclooctane, benzene, toluene or xylene.

7. A process according to one of claims 1 to 6, characterized in that the catalyst is made at temperatures of 20 to 120 °C, preferably 50 to 100 °C.

8. A catalyst system for the trimerization of acetylene and acetylene compounds, characterized by tetrakis(triphenylphosphite)nickel(O) and at least one further phosphite compound, selected from tris(2-isopropyl-5-methylphenyl)phosphite, tris(2,6-dimethylphenyl)phosphite, tris(2-tert.-butyl-5-methylphenyl)phosphite, tris-(2-sec.-butylphenyl)phosphite, tris(2-tert.-butyl-4-methylphenyl)-phosphite, tris(2,4-ditert.-butylphenyl)phosphite, tris(2-tert.-butylphenyl)-phosphite or tris(2-diphenylyl)phosphite.

9. A catalyst system according to claim 8, characterized by a molar ratio between tetrakis-(triphenylphosphite)nickel(O) and the further phosphite compound of 1 : 1 to 1 : 5, preferably 1 : 1.5 to 1 : 2.5.

## Revendications

1. Procédé de trimérisation de l'acétylène et des dérivés acétyléniques en benzène ou en dérivés benzéniques correspondants, caractérisé en ce que l'on fait réagir l'acétylène ou des dérivés acétyléniques à l'aide d'un catalyseur en tétrakis (triphénylphosphito)nickel (o) et à base d'au moins un autre dérivé phosphité à une température de 20 à 120 °C, éventuellement dans un solvant inerte.

2. procédé selon la revendication 1, caractérisé en ce que l'on met en oeuvre comme dérivés acétyléniques, le méthylacétylène, le phénylacétylène, le 3-méthylbut-3en-1-yn, le 4-méthoxybut-3-en-1-yn, l'alcool propargylique, l'éther éthyl propargylique, le 3-méthylbut-1-yn-3-ol, le 3-méthylpent-l-yn-3-ol ou le but-2-yn-1,4-diol.

3. Procédé selon les revendications 1 et 2, caractérisé en ce que l'on met en oeuvre comme dérivé phosphité supplémentaire le phosphite de tris(2-isopropyl-5-méthylphényle), le phosphite de tris(2,6-diméthylphényle), le phosphite de tris(2-terbutyl-5-méthylphényle), le phosphite de tris(2-sec-butylphényle), le phosphite de tris(2-terbutyl-4-méthylphényle), le phosphite de tris(2,4-didéterbutylphényle), le phosphite de tris(2-terbutylphényle) ou le phosphite de tris(2)diphénylyle).

4. Procédé selon l'une des revendications 1 à 3, caractérisé en ce que l'on effectue la réaction à une température de 50 à 100° C, de préférence de 70 à 90° C.

5. Procédé selon l'une des revendications 1 à 4, caractérisé en ce que l'on met en oeuvre le tétrakis (triphénylphosphito)nickel (o) et d'autres dérivés phosphités dans un rapport molaire allant de 1:1 à 1:5, de préférence de 1:1,5 à 1:2,5.

6. Procédé selon l'une des revendications 1 à 5, caractérisé en ce que l'on met en oeuvre pour l'obtention du catalyseur et/ou pour la triméri-sation des hydrocarbures aliphatiques, aromati-ques, et/ou araliphatiques comme de l'Hexane, du n-Octane, de l'Isooctane, du Cyclohexane, du Cyclooctane du Benzène, du Toluène, ou du xylène.

7. Procédé selon l'une des revendications 1 à 6, caractérisé en ce que l'on produit le catalyseur à des températures allant de 20 à 120° C, de préférence de 50 à 100° c.

8. Système de catalyseur pour la trimérisation de l'acétylène et des composés acétyléniques, caractérisé par du tétrakis (triphénylphosphito)-nickel (o) et au moins par un dérivé phosphité supplémentaire choisi dans le groupe constitué par le phosphite de tris(2-isopropyl-5-méthylphényle), le phosphite de tris(2,6-diméthylpéhnyle), le phosphite de tris(2-terbutyl-5-méthylphényle), le phosphite de tris(2-sec-butylphény-le), le phosphite de tris(2-terbutyl-4-méthylphényle), le phosphite de tris(2,4-diteutylphényle), le phosphite de tris(2-terbutylphényle), et le phosphite de tris(2-diphénylyle).

9. Système de catalyseur selon la revendication 8, caractérisé par un rapport molaire du tétrakis (triphénylphosphito)nickel (o) au dérivé phosphité supplémentaire allant de 1:1 à 1:5, de préférence 1:1,5 à 1:2,5.